# FASCICULE DE BREVET EUROPEEN

(11) **EP 2 944 304 B1**
(45) Date de publication et mention de la délivrance du brevet: **28.02.2018**
(21) Numéro de dépôt: 15167854.7
(22) Date de dépôt: 15.05.2015
(51) Int. Cl.: A61K 8/37, A61Q 1/10, A61K 8/06

(54) **COMPOSITION DE MAQUILLAGE POUR LES CILS**
SCHMINKZUSAMMENSETZUNG FÜR DIE WIMPERN
MAKE-UP COMPOSITION FOR THE EYELASHES

(30) Priorité: 15.05.2014 LU 92454
(43) Date de publication de la demande: 18.11.2015
(73) Titulaire: Luxcos S.A., 3225 Bettembourg (LU)
(72) Inventeur: Delas, Christophe, 3926 Mondercange (LU); Raso, Sophie, 57365 Ennery (LU)
(74) Mandataire: Office Freylinger

(56) Documents cités:
- EP-A1- 0 832 637
- WO-A1-2013/048919
- FR-A1- 2 954 156
- KR-A- 20110 036 297

## Description

### Domaine Technique

La présente invention concerne d'une manière générale une composition de maquillage des fibres kératiniques et en particulier les cheveux et les cils et plus particulièrement les cils.

### Etat de la technique

Les cils sont considérés comme un signe de beauté dans de nombreuses cultures, et, par voie de conséquence, les femmes recherchent continuellement à allonger leurs cils ou à les rendre plus épais. Parmi les voies qui permettent de réaliser cet objectif, nous citerons notamment les faux-cils ou encore les teintures de cils qui donnent une couleur plus marquée aux cils. Enfin, l'une des voies les plus couramment utilisée aujourd'hui est l'utilisation de mascara qui permet, de façon non permanente d'intensifier la longueur, la courbure ou encore l'épaisseur des cils.

Les cils sont divisés en deux parties : les cils supérieurs (de 150 à 200 cils en moyenne), les cils inférieurs moins nombreux (50-150 cils en moyenne). Les cils supérieurs sont généralement plus longs (8 à 12 mm) contre 6 à 8 mm pour les cils inférieurs.

La présente invention concerne plus particulièrement une composition de maquillage de type non permanente et en particulier des compositions de mascara. Ce type de composition a connu de nombreuses évolutions durant les 100 dernières années. Ce type de composition s'est tout d'abord présenté sous la forme d'une pâte faite de vaseline et de noir de charbon, puis sous la forme de pain ou « mascara cake ». Enfin, dans les années 50, les premiers mascaras dits « automatiques » ont fait leur apparition facilitant leur utilisation quotidienne. C'est ce type de mascara qui est utilisé aujourd'hui. Le mascara est l'un des produits cosmétiques les plus utilisés en cosmétique.

Les différentes propriétés nécessaires à une composition de mascara sont les suivantes :
- Une application aisée à l'aide d'une brosse type goupillon constituée de fibres de nylon® maintenues par un fil torsadé ou encore à l'aide d'une brosse de type « élastomère ». Nous entendrons par brosse de type « élastomère » les applicateurs de mascara issus de moulage thermoplastiques de matériaux synthétiques de différentes natures (HDPE, LDPE, dérivés siliconés, POM). Cette liste ne s'entendant pas être une liste exhaustive des techniques et des matériaux utilisés.
- Un effet rapide et immédiat. En effet, les consommatrices pressées ou non préfèrent généralement des formules ou plutôt des combinaisons brosses/formules qui procurent un effet rapide après quelques coups de brosse uniquement.
- Un temps de séchage qui n'est pas trop long et qui est surtout approprié au type d'effet recherché. Les formules de type « longlash » ou « curl » offrant une impression d'allongement ou de courbure des cils sèchent généralement plus rapidement que les formules dites « volume » pour lesquelles un temps de séchage plus long autorisera le retravail de la formule sur les cils pendant quelques minutes afin d'obtenir le résultat désiré.
- Une bonne tenue, nous différencierons ici deux types de tenue :
   ∘ Tout d'abord la tenue de la formule sur les cils. Le manque de tenue sur les cils se caractérise généralement soit par l'apparition de « flaking », c'est-à-dire de particules de petites tailles qui tombent des cils sur les pommettes ou encore par un phénomène appelé smudge. Ce phénomène se caractérise par le transfert de la formule des cils sur la peau qui entoure l'oeil. Dans ce cas, la région entourant l'oeil devient plus noir.
   ∘ Le second effet de tenue concerne plus particulièrement la tenue de l'effet. Quelque soit l'effet recherché par l'utilisation d'un mascara : longlash, curl ou volume, il est important que celui-ci se conserve au cours de la journée.

L'application du mascara dépend de nombreux facteurs ou paramètres physicochimiques. Parmi ces facteurs nous citerons :
- La viscosité de cisaillement de la composition généralement mesurée à l'aide d'un viscosimètre de type Brookfied et notée en mPa.s.
- La nature des cires mises en oeuvre au sein de la composition et leurs quantités respectives.
- La quantité des charges utilisées dans la composition
- L'applicateur et son affinité/compatibilité avec la composition ainsi que son taux de transfert vers les cils.

L'effet maquillage de la composition dépend directement de la synergie entre la composition et l'applicateur utilisé ainsi que de la manière dont la consommatrice applique la composition.

Le temps de séchage de la composition sur les cils dépend, quant à lui, de plusieurs facteurs :
- Le taux de matière sèche de la formule. Un taux de matière sèche important induira généralement un temps de séchage plus long.
- La viscosité de la formule. Les formules épaisses auront tendance à rendre le temps de séchage plus long.
- La quantité de composition appliquée sur les cils.

La tenue de la formule sur les cils dépend de la quantité de composition appliquée sur les cils mais aussi de la nature des ingrédients utilisés dans celle-ci. Les phénomènes de flaking pourront être évités par le développement de compositions qui ne sont pas trop dures après séchage, les cires trop dures utilisées en trop grandes proportions dans la formule seront ainsi évitées.

Les phénomènes de smudge seront écartés par la mise en oeuvre de composition dont la phase lipidique n'est pas en pourcentage trop important par rapport à la phase aqueuse.

La couleur de la composition dépend directement du ou des pigments utilisés dans la formulation mais elle dépendra aussi des ingrédients hydrophiles et hydrophobes mis en jeu au sein de la composition. L'homme de l'art sait en effet que certaines cires pourront apporter un effet gris à la composition. Leur utilisation en quantité trop importante sera donc évitée.

Compte tenu de la grande diversité d'ingrédients utilisés au sein de la formulation, le formulateur aura pour principal objectif le développement de compositions balancées qui possèdent les propriétés suivantes :
- Un temps de séchage adéquat
- Une application aisée
- Des effets immédiats marqués
- Une bonne tenue dans le temps, sans « flaking » ni « smudge »

### Objet de l'invention

Un objet de la présente invention est par conséquent de proposer une formulation de compositions cosmétiques ou de maquillage applicables sur les fibres kératiniques, en particulier les cils humains (ou même les faux-cils), qui permettent une tenue améliorée en évitant notamment le phénomène de « flaking ».

Conformément à l'invention, cet objectif est atteint par une composition selon la revendication 1, un procédé de préparation selon la revendication 11, respectivement une utilisation selon la revendication 12.

### Description générale de l'invention

Afin de résoudre le problème mentionné ci-dessus, la présente invention propose une composition de maquillage pour fibres, en particulier de fibres kératiniques, comprenant au moins une phase lipidique, au moins une phase aqueuse, au moins un pigment, au moins un agent filmogène et (une quantité efficace d')au moins un agent d'amélioration de la tenue. Selon l'invention, le ou un des agent(s) d'amélioration de la tenue est le Trimethyl Pentanyl Diisobutyrate. Un Trimethyl pentanyl Diisobutyrate utilisable est par exemple celui commercialisé par la société Eastman sous la dénomination Eastman TXIB.

En effet, les inventeurs ont découvert d'une manière surprenante que l'utilisation de Trimethyl Pentanyl Diisobutyrate permet de diminuer de manière significative le phénomène de « flaking » sans augmenter le phénomène de « smudge ». Cette amélioration se caractérise notamment par une tenue de la composition sur les cils améliorée dans le temps. Il est important de noter que cette tenue améliorée n'est pas obtenue au détriment du démaquillage qui reste conforme aux standards du métier.

Par l'expression « quantité efficace d'agent d'amélioration de la tenue », on entend une quantité suffisante pour obtenir une amélioration notable et significative de l'absence de « flaking » après application de la composition. Cette quantité minimale en agent d'amélioration de la tenue à mettre en oeuvre, qui peut varier selon la nature du type de composition retenue (émulsion huile/eau, émulsion eau/huile), ainsi qu'en fonction des autres ingrédients choisis, peut être déterminée par des essais et par la réalisation de panels de consommatrices. En pratique, la quantité totale d'agent(s) d'amélioration de la tenue représente en général 0.01 à 10% en poids, de préférence 0.05 à 5% en poids, en particulier de 0.1 à 4% en poids de la composition totale.

L'ajout de Trimethyl Pentanyl Diisobutyrate qui évite la formation de « flaking » au cours de la journée selon l'invention n'affecte pas négativement les autres qualités des compositions à savoir l'application, le temps de séchage ou encore les effets attendus (effets longlash, curl et volume).

Un avantage additionnel important de la présente invention est que les compositions de maquillage pour fibres à faible « flaking » peuvent comprendre les ingrédients habituellement utilisés par l'homme de l'art. L'homme de métier n'est donc pas obligé de reconsidérer tous les ingrédients et leurs effets. Le Trimethyl Pentanyl Diisobutyrate présente en effet une excellente compatibilité avec les ingrédients couramment utilisés dans ce domaine.

Les formulations selon l'invention se présentent sous la forme d'émulsions. Une émulsion est généralement un mélange stable de deux liquides qui dans les conditions normales ne sont pas miscibles. Deux types d'émulsions sont envisageables : les émulsions huile dans eau (H/E) et les émulsions eau dans huile (E/H). Les émulsions H/E forment généralement des compositions qui sont peu résistantes à l'eau sous réserve d'ajout d'ingrédients additionnels spécifiques. Les émulsions E/H forment quant à elles des compositions fortement résistantes à l'eau décrites même comme « waterproof » par l'homme de l'art.

Dans une variante, la composition de maquillage pour fibres kératiniques comprend en plus au moins une phase aqueuse et représente une émulsion de type H/E comprenant entre 30% et 80% en poids, de préférence entre 40% et 70% en poids d'eau par rapport à la composition totale. Dans ce type de formulations, l'eau est l'un des constituants majoritaires.

Dans une autre variante, la composition de maquillage selon l'invention comprend également en plus au moins une phase aqueuse, mais représente une émulsion de type eau dans huile (E/H) qui comprend entre 50% et 95% en poids, de préférence entre 60% et 90% en poids de composant lipidique par rapport à la composition totale.

Ces différentes compositions de maquillage pour les fibres kératiniques peuvent contenir le cas échéant les ingrédients ou types d'ingrédients suivants :

Des plastifiants, notamment :
- l'Acetyl Tributyl Citrate commercialisé sous la dénomination Citrofol B-II.
- l'Acetyl Triethyl Citrate commercialisé sous la dénomination Citroflex A-2.
- l'Acetyl Triethylhexyl citrate.

Des solvants ou des cosolvants. Ces cosolvants ont plusieurs fonctions au sein de la formulation selon l'invention. Ils permettent de solubiliser des ingrédients qui auraient une faible solubilité dans l'eau. Ils permettent également d'accélérer le temps de séchage de la composition. Parmi ces cosolvants qui ont la particularité d'être miscibles à l'eau, on citera par exemple l'éthanol, l'isopropanol, les glycols comme le propylène glycol, l'éthylène glycol, le 1,2-butylène glycol ou le dipropylène glycol.

Des cires. On considérera les cires comme des corps lipidiques solides à température ambiante (25°C), et qui seront miscibles dans des huiles en les portant à l'état liquide, en les chauffant par exemple. Les cires peuvent être d'origines variées :
- Les cires d'origine animale comme la cire d'abeille et les cires d'abeille modifiées ou encore la cire de lanoline.
- Les cires d'origine végétale, comme la cire de carnauba, la cire de candelilla, la cire de son de riz, la cire de berry, la cire d'alfa, la cire de myrthe, la cire de shellac, la cire du Japon, la cire d'orange, la cire de citron.
- Les cires d'origine minérale comme la cire de paraffine, la cire microcristalline, l'ozokérite, la cérésine.
- Les cires d'origine synthétique comme les cires de polyéthylène, les cires obtenues par synthèse de Fisher-Tropsch, les cires de silicone, les cires fluorées.

D'une manière générale, une composition selon l'invention peut contenir une teneur en cire allant jusqu'à 45% en poids, généralement entre 10% et 40% et de préférence entre 15% et 35% en poids de la composition totale.

Des corps gras cireux :
- Les alcools gras comme l'alcool cétylique, l'alcool cétéarylique, l'alcool stéarique, l'alcool oléique ou l'octyldodécanol.
- Les dérivés de l'acide stéarique comme le stéarate de glycéryle, les stéarates de polyéthylène glycol, ou tout autre type de dérivé obtenu par réaction d'estérification de la fonction acide carboxylique de l'acide stéarique.
- Les dérivés de rosinate comme le rosinate hydrogéné de glycéryle, le rosinate hydrogéné de méthyle.
- Les acides gras comme l'ester de la glycérine et d'acides gras en C₁₈-C₃₆, les esters d'olive hydrogénés.
- Les beurres. On citera par exemple le beurre de karité.

Les corps gras cireux peuvent être présents dans la composition à des teneurs comprises entre 0.1 % et 15% et de préférence à des teneurs comprises entre 0.5% et 10% de la composition totale.

Des huiles volatiles ou non volatiles peuvent être contenues dans des proportions comprises entre 0.1% et 15% et de préférence entre 0.1% et 7% en poids de la composition totale :
- Huiles volatiles comme les diméthicones ou les cyclométhicones, l'isododécane, l'isohexadécane ou l'isodécane.
- Huiles non volatiles comme l'huile de paraffine, l'huile de ricin, l'huile de ricin hydrogénée, les dérivés estérifiés d'olive, le polydécène hydrogéné, le propylène glycol, comme les esters de Guerbet (isononanoate de isotridécyle, neopentanoate d'isostéaryle, néopentanoate de tridécyle, octanoate de cétyle), les huiles silicones non volatiles (amodiméthicone, phényltriméthicone, phényltrimethicone).

Des filmogènes. Parmi ces filmogènes, nous citerons :
- Les polymères d'ester vinylique comme par exemple le polyvinylacétate, ou encore les copolymères d'esters vinyliques.
- Les copolymères de polyvinylpyrrolidone tels que les copolymères vinyl pyrrolidone et triacontane, les copolymères vinyl pyrrolidone et eicosene, et les copolymères vinyl pyrrolidone et hexadécène.
- Les polymères de cellulose et ses dérivés : sodium hydroxyéthylcellulose, carboxyméthylcellulose, cetyl hydroxyéthylcellulose, hydroxyéthylcellulose. Ces polymères sont commercialisés selon plusieurs grades qui se caractérisent notamment par des longueurs de chaines différentes.
- Les ingrédients décrits sous la dénomination polyquaternium. Ce sont des polymères cationiques qui ont des actions filmogènes mais peuvent aussi jouer le rôle d'agent antistatique.
- Les copolymères éthylène / propylène / styrène ou les copolymères butylène / éthylène / styrène contenus par exemple dans les ingrédients vendus sous la dénomination Versagel.
- Les polyesters obtenus par polycondensation d'acides dicarboxyliques avec des polyols en particulier des diols. Les diols et les acides dicarboxyliques peuvent être aromatiques, aliphatiques ou alicycliques.
- Les résines siloxysilicates comme le triméthylsiloxysilicate ou les copolymères de résines de silicone.
- Les polymères acryliques qui se présentent généralement sous la forme de dispersions aqueuses ou d'émulsions. Parmi ces polymères on citera les copolymères et homopolymères acryliques et les copolymères styrène / acryliques.
- Les polyuréthanes qui se différencient notamment par la nature des monomères qui les constituent. Ce type de polymère se présente également sous la forme d'émulsions ou de dispersions aqueuses.

Ces filmogènes peuvent être utilisés entre 0.5% et 25% en poids de la composition et de préférence entre 1% et 15% de la formulation.

Des pigments. Les pigments peuvent être de différentes natures et couleurs. On citera notamment les pigments inorganiques et les pigments organiques. Ces pigments sont susceptibles d'avoir subi un traitement particulier ou non. La teneur en pigment selon l'invention se situe généralement entre 0.1 et 15% et de préférence entre 1% et 12.5% en poids de la composition totale. Parmi ces pigments nous citerons notamment :
- Les pigments inorganiques :
   - Les oxydes de fer noirs, bruns, rouges et jaunes. Ces oxydes de fer se différencient notamment par leur degré d'oxydation ainsi que leur niveau d'hydratation.
   - Les oxydes de chrome et les hydrates de chrome qui permettent d'obtenir des nuances variables de verts.
   - Le bleu outremer.
   - Le bleu ferrique.
   - Le dioxyde de titane.
   - Le violet de manganèse.
- Les pigments organiques :
   - Le noir de carbone.
   - Les pigments de type D&C à base de baryum, strontium, calcium et aluminium ou encore le pigment obtenu à partir de la cochenille.

La composition selon l'invention peut être susceptible de comprendre des nacres. Les nacres sont des particules d'origine minérale synthétique de tailles variables et de couleurs et d'effets multiples. Les nacres sont constituées de substrats minéraux comme le mica naturel ou synthétique :
- fluorphlogopite synthétique.
- les borosilicates (calcium sodium borosilicate, calcium aluminium borosilicate).

Ces substrats sont recouverts de couches successives d'oxydes métalliques (oxydes de fer, oxyde de titane, oxydes de chrome) ou de pigments organiques.

Des charges. Les charges peuvent être minérales ou organiques, lamellaires ou sphériques. Les charges utilisées selon l'invention sont incolores ou blanches (non pigmentaires). Parmi les charges ont citera :
- Les charges minérales telles que le talc, le mica, la silice ou le kaolin.
- Les charges synthétiques comme les poudres de polyamide (Nylon®), les poudres de polymères de tétrafluoroéthylène (Téflon®), les particules de polyméthacrylate de méthyle, les poudres de polyuréthane.
- Les charges organiques comme la lauroyl-lysine, l'amidon et ses dérivés.

Les charges sont présentes en une teneur d'au moins 0.1% en poids par rapport au total de la composition et de préférence entre 0.1 % et 10% en poids de la composition.

Des fibres. Ces fibres peuvent notamment permettre d'améliorer l'effet allongeant de la formule. Les fibres ont une longueur pouvant aller de 0.01 mm à 10 mm et en particulier de 0.1 à 5 mm. Les fibres utilisables selon l'invention sont par exemple les fibres de polyamides (Nylon®).

Des agents gélifiants comme les homo- ou copolymères d'acide acrylique ou d'acide méthacrylique, les acides polyacryliques, le polyméthacrylate de sodium, les polymères de cellulose, les gommes arabiques, les gommes de xanthane. Ces différents gélifiants sont utilisés à des teneurs comprises entre 0.1% et 10% en poids de la composition et de préférence entre 0.1 % et 5% de la composition.

Des conservateurs. Ces ingrédients ont pour fonction de stabiliser la formule d'un point de vue bactériologique. Les conservateurs susceptibles de faire partie de ce type de formulation sont : le phénoxyéthanol, le propylparaben, le méthylparaben, le butylparaben, l'éthylparaben, l'isobutylparaben, le disodium EDTA, le tétrasodium EDTA, le déhydroacétate de sodium, le sorbate de potassium, l'acide benzoïque, le benzoate de sodium, l'imidazolidinylurée, l'alcool benzylique, la chlorophénésine. Les teneurs de ces ingrédients au sein de la composition suivront les prescriptions des réglementations cosmétiques en vigueur. Elles seront utilisées dans un pourcentage minimum à condition que ce pourcentage garantisse l'intégrité bactériologique de la composition dans des conditions normales d'utilisation.

Des antioxydants comme le BHT, le TBHQ, la vitamine E dans des teneurs comprises entre 0.05% et 0.3 %.

Des résines d'origine végétale comme la colophane, la shellac ou encore la résine élémi.

Des agents tensioactifs émulsionnants et des co-émulsionnants. Ces émulsionnants sont choisis de manière appropriée pour l'obtention de l'émulsion désirée. Ces agents tensioactifs peuvent être choisis parmi des agents non ioniques, anioniques, cationiques ou amphotériques. Plusieurs types d'émulsionnants peuvent être envisagés pour la réalisation de ce type d'émulsion :
- Les « savons » de l'acide stéarique ou de l'acide palmitique. Ces acides gras peuvent être neutralisés par la triéthanolamine, l'aminométhylpropanol, l'aminométhylpropanediol, l'hydroxyde de potassium ou encore l'hydroxyde de sodium.
- Les dérivés de phosphate comme le cetyl phosphate ou le cetyl phosphate de potassium.

Des actifs cosmétiques peuvent être utilisés dans la composition selon l'invention. Parmi ces actifs, on citera notamment les parfums, les émollients, les vitamines et les filtres solaires.

La composition selon l'invention peut être conditionnée dans un récipient hermétique. Ce même récipient comprend généralement un applicateur se présentant sous la forme d'une brosse comportant des poils maintenus par un fil torsadé ou sous la forme d'une brosse élastomère ou encore sous la forme d'un peigne, obtenus par moulage. Ces applicateurs sont généralement associés à une tige portée par l'élément de fermeture.

Les compositions applicables sur les fibres kératiniques comme les mascaras ont généralement un aspect pâteux ou crémeux dont la couleur dépend du pourcentage et de la nature du ou des matières colorantes mises en oeuvre.

La consistance de la formule est également dépendante des teneurs et de la nature des agents gélifiants, des charges, des cires ou encore des tensioactifs émulsionnants utilisés.

Dans un aspect supplémentaire, l'invention propose donc l'utilisation de Trimethyl Pentanyl Diisobutyrate comme agent d'amélioration de la tenue pour leurs effets positifs sur les phénomènes de « flaking » au cours de l'utilisation de la composition. De préférence, la quantité d'agent d'amélioration de la tenue représente en général de 0.01% à 10% en poids, de préférence 0.05 à 5% en poids, en particulier de 0.1 à 4% en poids de la composition totale.

L'invention concerne également un procédé de préparation d'une telle composition de maquillage des fibres kératiniques, notamment un mascara, comprenant le mélange d'au moins une phase lipidique, d'au moins un pigment, d'au moins un agent filmogène, de Trimethyl Pentanyl Diisobutyrate comme agent d'amélioration de la tenue, ainsi qu'au moins une phase aqueuse, et/ou éventuellement un ou plusieurs autres additifs choisis parmi les charges, les fibres, les agents gélifiants, les agents conservateurs, les antioxydants, les résines d'origine végétale, les agents tensioactifs émulsionnants et des co-émulsionnants, les parfums, les émollients, les vitamines et les filtres solaires.

Comme avantage supplémentaire de l'invention, en complément des avantages mentionnés ci-dessus en relation avec les compositions, il est à remarquer que le procédé ci-dessus peut être réalisé moyennant des installations tout à fait standard et ne demande pas de connaissances, ni de précautions particulières de l'opérateur.

En conclusion, les inventeurs ont découvert d'une manière surprenante que le Trimethyl Pentanyl Diisobutyrate permettaiet de réaliser l'objectif d'améliorer la tenue d'un mascara ou d'une composition de ce type.

Cette amélioration se caractérise notamment par une diminution significative du phénomène de « flaking » c'est-à-dire l'apparition au cours de la journée de particules noires ou de la couleur de la composition sur le visage dans la partie située en dessous de l'oeil. De préférence, le Trimethyl Pentanyl Diisobutyrate est ajouté au sein de la phase lipidique de la composition avant la réalisation de l'émulsion.

### Exemple 1 : Exemple de mascara crème

| | Cas A | Cas B | Cas C |
|---|---|---|---|
| | | selon l'invention | selon l'invention |
| **Phase A** | | | |
| Eau | 63.58 | 63.08 | 63.08 |
| Phenoxyethanol | 0.90 | 0.90 | 0.90 |
| Aminométhylpropanediol | 0.80 | 0.80 | 0.80 |
| Sorbate de potassium | 0.30 | 0.30 | 0.30 |
| Hydroxyéthylcellulose | 0.40 | 0.40 | 0.40 |
| Sodium déhydroacétate | 0.30 | 0.30 | 0.30 |
| PVP | 1.00 | 1.00 | 1.00 |
| Gomme arabique | 0.50 | 0.50 | 0.50 |

| **Phase B** | | | |
|---|---|---|---|
| Oxyde de fer noir | 10.00 | 10.00 | 10.00 |

| **Phase C** | | | |
|---|---|---|---|
| Acide stéarique | 3.50 | 3.50 | 3.50 |
| Cire de carnauba | 7.00 | 7.00 | 7.00 |
| Cire de paraffine | 7.00 | 7.00 | 7.00 |
| Cire de riz | 0.50 | 0.50 | 0.50 |
| Cire de thé | 0.50 | 0.50 | 0.50 |
| Glycéryl stéarate | 3.50 | 3.50 | 3.50 |

| **Phase D** | | | |
|---|---|---|---|
| Diméthicone | 0.20 | 0.20 | 0.20 |
| BHT | 0.02 | 0.02 | 0.02 |
| Acetyl tributyl citrate | | 0.25 | |
| Trimethyl Pentanyl Diisobutyrate | | 0.25 | 0.50 |

### Exemple 2 : Exemple de mascara waterproof

| | Cas A | Cas B | Cas C |
|---|---|---|---|
| | | selon l'invention | selon l'invention |
| | | | |

| **Phase A** | | | |
|---|---|---|---|
| Isododecane | 48.78 | 48.28 | 48.28 |
| Cire d'abeille | 15.50 | 15.50 | 15.50 |
| Cire de candelilla | 3.50 | 3.50 | 3.50 |
| Cire de carnauba | 2.50 | 2.50 | 2.50 |
| Antaron V 216 | 2.00 | 2.00 | 2.00 |
| Talc | 2.50 | 2.50 | 2.50 |
| BHT | 0.02 | 0.02 | 0.02 |
| Phenoxyethanol | 0.40 | 0.40 | 0.40 |
| Acetyl triethylhexyl Citrate | | 0.25 | |
| Trimethyl Pentanyl Diisobutyrate | | 0.25 | 0.50 |

| **Phase B** | | | |
|---|---|---|---|
| Oxyde de fer noir | 10.00 | 10.00 | 10.00 |

| **Phase C** | | | |
|---|---|---|---|
| Propylene glycol | 3.00 | 3.00 | 3.00 |
| Eau | 10.00 | 10.00 | 10.00 |
| Sorbate de potassium | 0.30 | 0.30 | 0.30 |
| Triethanolamine | 1.10 | 1.10 | 1.10 |
| Phenoxyethanol | 0.40 | 0.40 | 0.40 |

## Revendications

1. Composition de maquillage pour fibres, en particulier de fibres kératiniques, comprenant au moins une phase lipidique, au moins un pigment, au moins un agent filmogène, au moins une phase aqueuse et une quantité efficace d'un agent d'amélioration de la tenue, **caractérisé en ce que** l'agent d'amélioration de la tenue est le Trimethyl Pentanyl Diisobutyrate.

2. Composition selon la revendication 1, dans laquelle l'agent d'amélioration de la tenue représente 0.01% à 10% en poids, de préférence 0.05% à 5% en poids, en particulier de 0.1 à 4% en poids de la composition totale.

3. Composition selon l'une des revendications 1 ou 2, qui est une émulsion de type huile dans eau comprenant entre 30% et 80% en poids, de préférence entre 40% et 70% en poids, d'eau par rapport à la composition totale.

4. Composition selon l'une des revendications 1 ou 2, qui est une émulsion de type eau dans huile comprenant entre 50% et 95% en poids, de préférence entre 60% et 90% en poids, de composant lipidique par rapport à la composition totale.

5. Composition selon l'une quelconque des revendications 1 à 4, dans laquelle le au moins un pigment est choisi parmi les pigments inorganiques, notamment les oxydes de fer noirs, bruns, rouges et jaunes, les oxydes de chrome et les hydrates de chrome, le bleu outremer, le bleu ferrique, le dioxyde de titane, le violet de manganèse; les pigments organiques, notamment le noir de carbone, les pigments de type D&C à base de baryum, strontium, calcium et aluminium ou encore le pigment obtenu à partir de la cochenille; et les nacres de mica naturel ou synthétique, éventuellement recouvert de dioxyde de titane, d'oxyde de fer; ainsi que leurs combinaisons.

6. Composition selon l'une quelconque des revendications 1 à 5, dans laquelle le au moins un pigment représente de 0.1 à 15% en poids de la composition totale.

7. Composition selon l'une quelconque des revendications 1 à 6, dans laquelle le au moins un agent filmogène est choisi parmi les polymères d'ester vinylique comme par exemple le polyvinylacétate, ou encore les copolymères d'esters vinyliques; les copolymères de polyvinylpyrrolidone tels que les copolymères vinylpyrrolidone et triacontane, les copolymères vinyl pyrrolidone et éicosène, et les copolymères vinylpyrrolidone et hexadécène; les polymères de cellulose et ses dérivés : sodium hydroxyéthylcellulose, carboxyméthylcellulose, cétyl hydroxyéthylcellulose, hydroxyéthylcellulose; les polyquaterniums; les copolymères éthylène/propylène/styrène ou les copolymères butylène/éthylène/styrène; les polyesters obtenus par polycondensation d'acides dicarboxyliques avec des polyols en particulier des diols; les résines siloxysilicates comme le triméthylsiloxysilicate ou les copolymères de résines de silicone;

8. Composition selon l'une quelconque des revendications 1 à 7, dans laquelle le au moins un agent filmogène est choisi parmi les polymères acryliques sous forme de dispersions aqueuses ou d'émulsions, notamment les copolymères et homopolymères acryliques et les copolymères styrène/acryliques; les polyuréthanes sous forme d'émulsions ou de dispersions aqueuses; ainsi que leurs mélanges.

9. Composition selon l'une quelconque des revendications 1 à 8, dans laquelle le ou les agent(s) filmogène(s) représente(nt) entre 0.5% et 25% en poids, de préférence entre 1% et 15% en poids de la composition totale.

10. Composition selon l'une quelconque des revendications 1 à 9, comprenant en outre un ou plusieurs autres additifs choisis parmi les charges, les fibres, les agents gélifiants, les agents conservateurs, les antioxydants, les résines d'origine végétale, les agents tensioactifs émulsionnants et des co-émulsionnants, les parfums, les émollients, les vitamines et les filtres solaires.

11. Procédé de préparation d'une composition de maquillage pour fibres, en particulier de fibres kératiniques, selon l'une quelconque des revendications 1 à 10, comprenant le mélange d'au moins une phase lipidique, d'au moins un pigment, d'au moins un agent filmogène, d'au moins une phase aqueuse, et de Trimethyl Pentanyl Diisobutyrate, ainsi qu'éventuellement un ou plusieurs autres additifs choisis parmi les charges, les fibres, les agents gélifiants, les agents conservateurs, les antioxydants, les résines d'origine végétale, les agents tensioactifs émulsionnants et des co-émulsionnants, les parfums, les émollients, les vitamines et les filtres solaires.

12. Utilisation de Trimethyl Pentanyl Diisobutyrate comme agent d'amélioration de la tenue pour améliorer la tenue d'un maquillage pour fibres sur ces fibres, en particulier pour réduire l'effet de « flaking ».

## Patentansprüche

1. Schminkzusammenzusetzung für Fasern, insbesondere Keratinfasern, welche mindestens eine Lipidphase, mindestens ein Pigment, mindestens einen Filmbildner, mindestens eine Wasserphase sowie eine wirksame Menge eines Fixierungsverbesserers umfasst, **dadurch gekennzeichnet, dass** es sich bei dem Fixierungsverbesserer um Trimethylpentanyldiisobutyrat handelt.

2. Zusammensetzung nach Anspruch 1, wobei der Fixierungsverbesserer 0,01 % bis 10 % nach Gewicht, vorzugsweise 0,05 % bis 5 % nach Gewicht, insbesondere 0,1 bis 4 Gewichts-% der Zusammensetzung insgesamt ausmacht.

3. Zusammensetzung nach einem der Ansprüche 1 oder 2, wobei es sich um eine Emulsion vom Typ Öl-in-Wasser handelt, die zwischen 30 % und 80 % nach Gewicht, vorzugsweise zwischen 40 % und 70 % nach Gewicht, an Wasser umfasst, bezogen auf die Zusammensetzung insgesamt.

4. Zusammensetzung nach einem der Ansprüche 1 oder 2, wobei es sich um eine Emulsion vom Typ Wasser-in-Öl handelt, die zwischen 50 % und 95 % nach Gewicht, vorzugsweise zwischen 60 % und 90 % nach Gewicht, an Lipidbestandteil umfasst, bezogen auf die Zusammensetzung insgesamt.

5. Zusammensetzung nach einem beliebigen der Ansprüche 1 bis 4, wobei das mindestens eine Pigment aus den anorganischen Pigmenten, insbesondere den schwarzen, braunen, roten und gelben Eisenoxiden, den Chromoxiden und den Chromhydraten, Ultramarinblau, Berliner Blau, Titandioxid, Manganviolett; den organischen Pigmenten, insbesondere Ruß, den Pigmenten des Typs D&C auf Grundlage von Barium, Strontium, Calcium und Aluminium oder auch dem Pigment, welches aus Cochenilleschildläusen gewonnen wird; und den natürlichen oder synthetischen Glimmerperlglanzmitteln, wobei möglicherweise ein Überzug aus Titandioxid, Eisenoxid vorliegt, sowie Kombinationen davon ausgewählt ist.

6. Zusammensetzung nach einem beliebigen der Ansprüche 1 bis 5, wobei das mindestens eine Pigment 0,1 bis 15 Gewichts-% der Zusammensetzung insgesamt ausmacht.

7. Zusammensetzung nach einem beliebigen der Ansprüche 1 bis 6, wobei der mindestens eine Filmbildner aus den Vinylesterpolymeren, wie beispielsweise Polyvinylacetat, oder auch den Vinylestercopolymeren; den Polyvinylpyrrolidon-Copolymeren wie etwa den Copolymeren von Vinylpyrrolidon und Triacontan, den Copolymeren von Vinylpyrrolidon und Eicosen und den Copolymeren von Vinylpyrrolidon und Hexadecen; den Polymeren von Cellulose und deren Derivaten: Natrium-Hydroxyethylcellulose, Carboxymethylcellulose, Cetyl-Hydroxyethylcellulose, Hydroxyethylcellulose; den Polyquaternium-Mitteln; den Ethylen/Propylen/Styrol-Copolymeren oder den Butylen/Ethylen/Styrol-Copolymeren; den Polyestern, die durch Polykondensation von Dicarbonsäuren mit Polyolen, insbesondere Diolen, erhalten werden; den Siloxysilikatharzen wie etwa Trimethylsiloxysilikat oder den Copolymeren von Silikonharzen ausgewählt ist.

8. Zusammensetzung nach einem beliebigen der Ansprüche 1 bis 7, wobei der mindestens eine Filmbildner aus den Acrylsäurepolymeren in Form von wässrigen Dispersionen oder Emulsionen, insbesondere den Acrylsäurecopolymeren und -homopolymeren und den Styrol/AcrylsäureCopolymeren; den Polyurethanen in Form von Emulsionen oder wässrigen Dispersionen; sowie den Mischungen davon ausgewählt ist.

9. Zusammensetzung nach einem beliebigen der Ansprüche 1 bis 8, wobei der oder die Filmbildner zwischen 0,5 % und 25 % nach Gewicht, vorzugsweise zwischen 1 % und 15 % nach Gewicht der Zusammensetzung insgesamt ausmachen.

10. Zusammensetzung nach einem beliebigen der Ansprüche 1 bis 9, welche darüber hinaus einen oder mehrere Zusatzstoffe umfasst, die aus den Füllstoffen, den Fasern, den Gelbildnern, den Konservierungsstoffen, den Antioxidantien, den Harzen pflanzlicher Herkunft, den Tensiden, welche als Emulgator oder Co-Emulgator wirken, den Duftstoffen, den Erweichungsmitteln, den Vitaminen und den Sonnenschutzfiltern ausgewählt sind.

11. Verfahren zur Herstellung einer Schminkzusammensetzung für Fasern, insbesondere Keratinfasern, nach einem beliebigen den Ansprüche 1 bis 10, welches das Vermischen mindestens einer Lipidphase, mindestens eines Pigments, mindestens eines Filmbildners, mindestens einer Wasserphase und von Trimethylpentanyldiisobutyrat sowie möglicherweise eines oder mehrerer Zusatzstoffe umfasst, wobei letztere aus den Füllstoffen, den Fasern, den Gelbildnern, den Konservierungsstoffen, den Antioxidantien, den Harzen pflanzlicher Herkunft, den Tensiden, welche als Emulgator oder Co-Emulgator wirken, den Duftstoffen, den Erweichungsmitteln, den Vitaminen und den Sonnenschutzfiltern ausgewählt sind.

12. Verwendung von Trimethylpentanyldiisobutyrat als Fixierungsverbesserer, um die Fixierung eines Schminkmittels für Fasern auf diesen Fasern zu verbessern, insbesondere um den "Abschuppungs"-Effekt zu verringern.

## Claims

1. A make-up composition for fibres, in particular keratin fibres, comprising at least one lipid phase, at least one pigment, at least one film-forming agent, at least one aqueous phase and an effective quantity of a durability-enhancing agent, **characterised in that** the durability-enhancing agent is Trimethyl Pentanyl Diisobutyrate.

2. A composition according to claim 1, in which the durability-enhancing agent represents 0.01% to 10% by weight, preferably 0.05% to 5% by weight and in particular 0.1 to 4% by weight of the total composition.

3. A composition according to one of claims 1 or 2, which is an oil-in-water type emulsion comprising between 30% and 80% by weight, preferably between 40% and 70% by weight, of water relative to the total composition.

4. A composition according to one of claims 1 or 2, which is a water-in-oil type emulsion comprising between 50% and 95% by weight, preferably between 60% and 90% by weight, of lipid component relative to the total composition.

5. A composition according to any one of claims 1 to 4, in which the at least one pigment is selected from among inorganic pigments, in particular black, brown, red and yellow iron oxides, chromium oxides and chromium hydrates, ultramarine blue, ferric blue, titanium dioxide or manganese violet; organic pigments, in particular carbon black, D&C type pigments based on barium, strontium, calcium and aluminium or alternatively the pigment obtained from cochineal; and natural or synthetic mica pearlescents, optionally covered with titanium dioxide or iron oxide; together with the combinations thereof.

6. A composition according to any one of claims 1 to 5, in which the at least one pigment represents from 0.1 to 15% by weight of the total composition.

7. A composition according to any one of claims 1 to 6, in which the at least one film-forming agent is selected from among vinyl ester polymers such as, for example polyvinyl acetate, or alternatively vinyl ester copolymers; polyvinylpyrrolidone copolymers such as vinylpyrrolidone and triacontane copolymers, vinylpyrrolidone and eicosene copolymers, and vinylpyrrolidone and hexadecene copolymers; cellulose polymers and the derivatives thereof: sodium hydroxyethylcellulose, carboxymethylcellulose, cetyl hydroxyethylcellulose, hydroxyethylcellulose; polyquaterniums; ethylene/propylene/styrene copolymers or butylene/ethylene/styrene copolymers; polyesters obtained by polycondensing dicarboxylic acids with polyols, in particular diols; siloxysilicate resins such as trimethylsiloxysilicate or silicone resin copolymers.

8. A composition according to any one of claims 1 to 7, in which the at least one film-forming agent is selected from among acrylic polymers in the form of aqueous dispersions or emulsions, in particular acrylic copolymers and homopolymers and styrene/acrylic copolymers; polyurethanes in the form of emulsions or aqueous dispersions; as well as mixtures thereof.

9. A composition according to any one of claims 1 to 8, in which the film-forming agent(s) represent(s) between 0.5% and 25% by weight, preferably between 1% and 15% by weight of the total composition.

10. A composition according to any one of claims 1 to 9, furthermore comprising one or more other additives selected from fillers, fibres, gelling agents, preservatives, antioxidants, resins of plant origin, emulsifying and co-emulsifying surfactants, perfumes, emollients, vitamins and sunscreens.

11. A method for preparing a make-up composition for fibres, in particular keratin fibres, according to any one of claims 1 to 10, comprising mixing at least one lipid phase, at least one pigment, at least one film-forming agent, at least one aqueous phase, and Trimethyl Pentanyl Diisobutyrate, optionally together with one or more other additives selected from fillers, fibres, gelling agents, preservatives, antioxidants, resins of plant origin, emulsifying and co-emulsifying surfactants, perfumes, emollients, vitamins and sunscreens.

12. Use of Trimethyl Pentanyl Diisobutyrate as a durability-enhancing agent to enhance the durability of a fibre make-up on said fibres, in particular to reduce the "flaking" effect.
